# FASCICULE DE BREVET EUROPEEN

(11) **EP 0 625 166 B1**
(45) Date de publication et mention de la délivrance du brevet: **10.09.1997**
(21) Numéro de dépôt: 93904154.7
(22) Date de dépôt: 04.02.1993
(51) Int. Cl.: C08B 37/10, A61K 31/725

(54) **POLYSACCHARIDES SULFATES, PROCEDE DE PREPARATION, COMPOSITION PHARMACEUTIQUE ET UTILISATION**
SULFATIERTE POLYSACCHARIDE, VERFAHREN ZU DEREN HERSTELLUNG, PHARMAZEUTISCHE ZUSAMMENSETZUNG UND IHRE VERWENDUNG
SULPHATED POLYSACCHARIDES, PREPARATION THEREOF, PHARMACEUTICAL COMPOSITION AND USE THEREOF

(30) Priorité: 07.02.1992 FR 9201383
(43) Date de publication de la demande: 23.11.1994
(73) Titulaire: RHONE-POULENC RORER S.A., 92160 Antony (FR)
(72) Inventeur: UZAN, André, F-75116 Paris (FR)
(74) Mandataire: Becker, Philippe
(86) Numéro de dépôt international: FR9300114
(87) Numéro de publication internationale: WO9316112

(56) Documents cités:
- EP-A- 0 337 327
- DE-A- 2 945 595
- FR-A- 2 622 450
- FR-A- 2 663 639
- CARBOHYDRATE RESEARCH, Vol. 141, 1985, pages 121-136
- THROMBOSIS AND HAEMOSTASIS, Vol. 61, No. 1, 1989, pages 30-34

## Description

La présente invention concerne le domaine des polysaccharides de faible masse moléculaire. Plus particulièrement, elle concerne des compositions oligosaccharidiques présentant d'excellentes propriétés pharmacologiques et antithrombotiques.

D'une manière générale, les traitements antithrombotiques font appel à deux grandes catégories d'agents, à savoir les agents anticoagulants et les agents antiplaquettaires.

Parmi les agents anticoagulants, les composés anti-vitamine K constituent une famille très importante. Ces composés étant actifs par voie orale, ils sont utilisés dans de nombreuses indications. Cependant, leur emploi est encore limité par certains inconvénients et notamment les risques d'hémorragies qu'ils occasionnent et la difficulté d'adapter la posologie à un traitement de longue durée.

Les héparines constituent la seconde catégorie d'agents anticoagulants. Ce sont des substances biologiques d'extraction de la famille des glycosaminoglycans composées d'oligosaccharides ayant des longueurs de chaînes et des degrés de sulfatation variables. Plus précisément, les masses moléculaires des chaînes constituant l'héparine non fractionnée se répartissent généralement entre 2000 et 40000 daltons. Les héparines sont utilisées dans différents types de thromboses, notamment dans le traitement ou la prévention des thromboses veineuses, éventuellement associées à d'autres thérapeutiques.

L'inconvénient des héparines réside dans leur activité anticoagulante élevée qui peut engendrer des hémorragies, et dans leur sensibilité à certains facteurs inhibiteurs du sang tels que le facteur 4 plaquettaire, qui impose l'utilisation de doses relativement importantes.

Par ailleurs, les héparines sont des produits très hétérogènes. Il est donc difficile d'évaluer leur mécanisme d'action, d'apprécier la contribution de chacune des composantes dans l'activité globale de l'héparine, et, de ce fait, d'augmenter l'activité antithrombotique sans augmenter les effets secondaires. L'évolution des connaissances scientifiques dans ce domaine a révélé que les propriétés biologiques, c'est-à-dire les effets sur la coagulation sanguine, de même que les propriétés pharmacologiques, c'est à dire les activités antithrombotiques in vivo, de ces oligosaccharides varient en fonction de leur masse moléculaire.

Une première solution aux inconvénients mentionnés plus haut a été apportée par les héparines de faible masse moléculaire. Ces héparines sont des mélanges d'oligosaccharides sulfatés dont les masses moléculaires se répartissent généralement entre 2000 et 10000 daltons. Ces héparines sont obtenues par fractionnement des chaînes oligosaccharidiques de l'héparine (par exemple par gel-perméation selon Barrowcliffe et al., Thrombosis research 12 (1977) 27-36), ou par fragmentation (dépolymérisation) des chaînes oligosaccharidiques de l'héparine au moyen d'agents chimiques ou enzymatiques. En particulier, la dépolymérisation a été décrite par traitement d'un ester d'héparine en présence d'une base forte (EP 40144). Elle peut également être réalisée par traitement d'héparine en présence d'acide nitreux, ou par action d'une héparinase (EP 64452). Ces différents procédés conduisent à des mélanges d'oligosaccharides ayant la structure générale des polysaccharides constitutifs de l'héparine, mais ayant un poids moléculaire moyen en poids inférieur. Ces modifications se traduisent généralement par une meilleure biodisponibilité et un effet moindre sur le saignement par rapport à l'héparine non fractionnée. Des produits obtenus par séparation de ces mélanges en une fraction de poids moléculaire moyen de 3300 daltons et une fraction de poids moléculaire moyen de 6500 daltons ont également été testés (S. BEGUIN et coll., Thromb. And Haemost., 61(I), 30-34 (1989).

Plus particulièrement, les recherches se sont essentiellement orientées vers des mélanges dérivés de l'héparine ayant des chaines oligosaccharidiques très courtes. Ainsi, le brevet EP 27089 indique que des mélanges d'oligosaccharides dérivés de l'héparine ne renfermant pas plus de 8 motifs saccharidiques possédent une activité spécifique antithrombotique supérieure à l'héparine. De la même manière, des hexasaccharides ont été préparés et leurs propriétés antithrombotiques étudiées (EP 64452). On peut encore citer les brevets plus récents EP 84 999 et EP 301 618 sur des polysaccharides tels que des hexa-, des penta- et des tétrasaccharides dérivés de l'héparine.

Cependant, les produits décrits jusqu'à aujourd'hui n'ont pas permis de résoudre de manière totalement satisfaisante les problèmes rencontrés avec les héparines. En particulier, les héparines de faible masse moléculaire ont pour la plupart une action inhibitrice vis-à-vis de la thrombine (activité anti IIa) plus de 6 fois inférieure à celle de l'héparine. Expérimentalement, sur un modèle de thrombose classique tel celui de Wessler chez le lapin, les doses antithrombotiques sont très supérieures à celles de l'héparine.

La demanderesse a maintenant montré qu'il est possible d'obtenir, à partir d'héparines natives ou dépolymérisées, des mélanges d'oligosaccharides ayant d'excellentes propriétés biologique et pharmacologique, et par conséquent, de meilleures potentialités thérapeutiques.

De manière inattendue, la demanderesse a en effet trouvé que lorsque l'on réalise un mélange d'oligosaccharides dont la répartition moléculaire est caractérisée par une proportion égale ou supérieure à 70 % d'oligosaccharides sulfatés de masse moléculaire comprise entre 5400 et 7800 daltons et 5 % au moins d'oligosaccharides sulfatés de masse moléculaire supérieure à 6900 daltons, possédant une activité anti IIa supérieure à 60 UI/mg et, de préférence, égale ou supérieure à 70 UI/mg, cette préparation conserve les avantages de bonne biodisponibilité et d'effets négligeables sur le saignement des héparines de faible masse moléculaire, mais présente en outre l'avantage d'un pouvoir inhibiteur accru vis-à-vis de la thrombine, et d'une activité antithrombotique supérieure avec une durée d'action au moins égale.

Un objet de l'invention réside donc dans un mélange d'oligosaccharides sulfatés présentant la structure générale des oligosaccharides constitutifs de l'héparine, caractérisé en ce qu'il comprend au moins 70 % d'oligosaccharides ayant une masse moléculaire comprise entre 5400 et 7800 daltons, au moins 5 % d'oligosaccharides ayant une masse moléculaire supérieure à 6900 daltons, et en ce qu'il possède une activité anti IIa supérieure à 60 UI/mg et, de préférence, égale ou supérieure à 70 UI/mg.

Un tel mélange réunit pour la première fois les avantages de l'héparine non fractionnée et ceux des héparines de faible masse moléculaire.

Par rapport à l'héparine native, les mélanges de l'invention présentent en outre une sensibilité moins forte aux facteurs inhibiteurs du sang tels que le facteur 4 plaquettaire, ce qui augmente leur potentialité thérapeutique.

D'autres avantages des mélanges de l'invention résident notamment dans la réduction de certains effets secondaires indésirables, tels que l'effet thrombocytopénique. L'un des inconvénients des mélanges connus dérivés de l'héparine provient de la chute du nombre de plaquettes qu'ils peuvent occasionner. Cet effet indésirable est fortement diminué lorsque les mélanges de l'invention sont employés.

Les propriétés énoncées ci-dessus permettent une utilisation pharmacologique particulièrement performante, notamment dans la prophylaxie et le traitement des thromboses veineuses ou artérielles. En outre, elles devraient rendre possible l'utilisation de doses plus importantes in vivo sans accroître les risques hémorragiques.

Plus préférentiellement, les mélanges de l'invention comprennent au moins 10 % d'oligosaccharides ayant une masse moléculaire supérieure à 6900 daltons.

Par ailleurs, des mélanges préférés selon l'invention comprennent 70 % d'oligosaccharides ayant une masse moléculaire comprise entre 5700 et 7500 daltons.

Encore plus préférentiellement, les mélanges de l'invention comprennent au moins 60 % d'oligosaccharides ayant une masse moléculaire comprise entre 5700 et 6900 daltons.

Par ailleurs, l'activité anti IIa des mélanges de l'invention est de préférence inférieure à leur activité anti Xa.

Dans un mode préféré, les mélanges de l'invention sont plus particulièrement des fractions d'héparine dépolymérisée. Comme indiqué précédemment, l'héparine dépolymérisée peut être obtenue par toute technique chimique, enzymatique ou autre, connue de l'homme de l'art permettant de fragmenter les chaînes oligosaccharidiques de l'héparine. En particulier, les procédés décrits dans les brevets EP 40144, EP 64452, EP 37319 ou EP 337327 conviennent pour l'invention.

Encore plus préférentiellement, les mélanges de l'invention sont constitués d'oligosaccharides ayant un acide 2-O-sulfo 4 enopyranosuronique à l'une de leurs extrémités.

Un mélange particulièrement avantageux est constitué par une fraction d'héparine dépolymérisée par action d'une base sur un ester d'héparine.

Un autre objet de l'invention concerne un procédé de préparation d'un mélange tel que défini ci-avant caractérisé en ce que l'on fractionne une héparine ou une héparine dépolymérisée par gel-filtration comme indiqué à la revendication 10.

Le procédé de l'invention fait intervenir plusieurs paramètres, dont le contrôle permet de calibrer la masse et la répartition moléculaires du mélange final. Ces paramètres sont notamment la force ionique de l'éluant et la nature du support utilisé.

Le fractionnement est caractérisé en ce que l'on réalise successivement les étapes consistant à (i) mettre l'héparine ou l'héparine dépolymérisée de départ en solution dans l'éluant, (ii) faire passer la solution ainsi obtenue à travers une colonne au moins contenant le support solide pour la gel-filtration préalablement équilibrée avec le même éluant, et (iii) récupérer les fractions de poids moléculaire désiré.

Dans un mode particulier de mise en oeuvre de l'invention, on utilise comme héparine de départ une héparine dépolymérisée. Une telle héparine peut être obtenue selon les procédés décrits dans les brevets EP 40144, EP 64452, EP 37319 ou EP 337327.

Encore plus préférentiellement, on utilise une héparine dépolymérisée par action d'une base sur un ester d'héparine, comme par exemple selon la technique décrite dans le brevet EP 40 144. En particulier, la dépolymérisation peut être réalisée en milieu aqueux ou au sein d'un solvant organique inerte, sous l'action d'une base organique ou minérale telle que par exemple d'hydroxyde de sodium ou de potassium, d'un carbonate alcalin ou d'une amine tertiaire (triéthylamine, triéthylène-diamine, etc). L'action de la base sur l'ester permet de réaliser une dépolymérisation partielle et contrôlée de l'héparine sans altérer sa structure générale.

Comme éluant utilisable dans le procédé de l'invention, on peut citer différents types de solutions salines, telles que des solutions de chlorure de sodium. Cependant, la demanderesse a montré que, pour obtenir des fractions ayant les meilleures propriétés, il est particulièrement avantageux de réaliser le fractionnement en utilisant un éluant choisi parmi les tampons phosphate, tels que notamment phosphate de potassium, phosphate de sodium ou NH₄H₂PO₄. Il est encore possible d'utiliser des solutions de NaClO₄ ou de NH₄NO₃ qui permettent d'obtenir des mélanges ayant d'excellentes caractéristiques.

La concentration de l'éluant et donc sa force ionique sont adaptées au mélange final recherché. Notamment, la concentration de l'éluant est avantageusement inférieure à 1M, et, encore plus préférentiellement, comprise entre 0,1 et 0,5 M.

Lorsque l'on utilise un tampon phosphate, il est particulièrement avantageux d'opérer à des concentrations proches de 0,2 M.

Dans la seconde étape du procédé de l'invention, le support utilisé est généralement choisi en fonction de la masse moléculaire moyenne du mélange de départ (héparine native, dépolymérisée ..), du produit final recherché, et du comportement du mélange de départ dans l'éluant utilisé. Avantageusement, on utilise comme support un gel de type polyacrylamide-agarose. A titre d'exemple on peut citer les gels AcA 54, AcA 202, séphadex G25 ou G50, ou encore Biogel P30 qui donnent de bons résultats.

Dans un premier mode particulièrement avantageux de mise en oeuvre du procédé de l'invention, lors de la seconde étape du fractionnement, le support solide est réparti en plusieurs colonnes disposées en série. Cette variante de l'invention permet d'employer des quantités finales de support pour la gel-filtration importantes, sans les inconvénients de l'art antérieur, à savoir essentiellement les phénomènes de tassement. De cette manière, la séparation est beaucoup plus nette, y compris dans les hauts poids moléculaires, en une seule opération de fractionnement, et les supports sont plus facilement régénérables.

Le nombre de colonnes utilisées est adapté par l'homme de l'art en fonction du volume et de la nature du gel employé, de manière à obtenir le meilleur équilibre entre l'efficacité de la séparation et l'effet néfaste dû au tassement du gel.

Pour des raisons pratiques de mise en oeuvre, on préfère généralement utiliser dans la seconde étape du procédé un nombre de colonnes inférieur à 20.

A titre illustratif, 40 litres de gel AcA 202 peuvent être répartis en 10 colonnes de 4 litres ou 130 litres en 10 colonnes de 13 litres.

Dans un autre mode particulièrement avantageux de mise en oeuvre du procédé de l'invention, on utilise lors de la seconde étape du fractionnement successivement 2 types de supports au moins, ayant des caractéristiques de séparation différentes. Cette variante de l'invention permet d'obtenir un fractionnement final de meilleure qualité.

A titre d'exemple, le fractionnement peut être réalisé sur la séquence suivante de gels : AcA 202 - AcA 54 - AcA 202.

Pour une meilleure mise en oeuvre de l'invention, il est important d'employer des quantités élevées de gel, de manière à réaliser une séparation plus nette et à obtenir une plus grande homogénéité. Compte tenu toutefois des débits assez lents utilisés pour ce genre de gel-filtration, le volume de gel doit être adapté à la quantité de produit à séparer, de manière à obtenir le meilleur équilibre entre la séparation et l'effet de diffusion longitudinale.

Avantageusement, dans le procédé de l'invention, le rapport héparine de départ (g)/ volume de gel (1) est inférieur à 2, et encore plus préférentiellement compris entre 0,5 et 1,5.

Un autre objet de l'invention concerne une composition pharmaceutique ayant comme principe actif un mélange tel que défini ci-avant. Une telle composition peut être utilisée de manière particulièrement avantageuse dans la prophylaxie ou le traitement ou la prévention des accidents thrombotiques. Plus précisément, elle peut être utilisée:
- dans la prévention des thromboses veineuses dans les situations à risques,
- dans la prévention des accidents thrombotiques artériels, notamment dans le cas d'infarctus du myocarde,
- en régime post-opératoire, dans la prévention de thromboses veineuses chez les malades chirurgicaux, ou encore,
- dans la prévention des thromboses dans le matériel chirurgical.

La présente invention sera plus complètement décrite à l'aide des exemples qui suivent, qui doivent être considérés comme illustratifs et non limitatifs. Dans ces exemples, les caractéristiques physicochimiques, biologiques et pharmacologiques des mélanges ont été déterminées selon les techniques suivantes :

### Masse moléculaire et répartition moléculaire

Les masses moléculaires et les répartitions moléculaires des produits sont déterminées par chromatographie liquide haute pression avec deux colonnes en série, par exemple celles commercialisées sous la désignation TSK G3000 XL et TSK G2000 XL couplées avec un détecteur réfractométrique.

L'éluant utilisé est du nitrate de lithium 0,5M et le débit est de 0,6 ml/minute. Le système est calibré avec des étalons monodisperses obtenus par fractionnement de l'enoxaparine (Pharmuka) par chromatographie d'exclusion sur agarose-polyacrylamide (IBF) selon la technique décrite par Barrowcliffe et coll., Thromb. Res., 12, 27-36 (1977-78) ou D.A. Lane et coll., Thromb. Res. &é, 257-271 (1977-78). Les résultats sont calculés à l'aide du logiciel GPC6 (Perkin Elmer).

### Activité anti Xa

La technique utilisée est celle décrite par Teien A.N. et Lie M. dans Thrombosis research 10 (1977) 399-410, avec comme étalon le premier étalon international des héparines de bas poids moléculaire (WHO LMWH1).

### Activité anti IIa

La technique utilisée est celle décrite par Anderson L.O. et al., dans Thrombosis research 15 (1979) 531-541, avec comme étalon le premier étalon international des héparines de bas poids moléculaire (WHO LMWH1).

### APTT

La technique utilisée est la technique "Actimat" (Bio Mérieux) telle que décrite par Bell W.N. et Alton H.G. dans Nature 174 (1954) 880-881.

### Thrombose veineuse chez le lapin

La technique utilisée est celle décrite par Wessler S. dans Journal of Clinical investigation 34 (1955) 647-651.

### Temps de saignement chez le rat

La technique utilisée est celle décrite par Palm M. et al., dans Thrombosis and haemostasis 64(1) (1990) 127-132.

### Légende des figures :

Figure 1 : Activité APTT in vitro sur plasma humain des mélanges de l'invention comparée à d'autres héparines.

Figure 2 (a et b) : Cinétique de l'activité anti Xa des mélanges de l'invention chez le lapin : comparaison avec une autre héparine de faible masse moléculaire.

Figure 3 (a et b) : Cinétique de l'activité anti IIa des mélanges de l'invention chez le lapin : comparaison avec une autre héparine de faible masse moléculaire.

### Exemple 1 : Préparation d'une héparine dépolymérisée.

A une solution de 10 g d'héparinate de sodium dans 100 ml d'eau on ajoute une solution de 25 g de chlorure de benzéthonium dans 125 ml d'eau. Le produit obtenu à température ambiante est filtré, lavé à l'eau puis séché. 15 g d'héparinate de benzéthonium ainsi obtenu sont mis en solution dans 75 ml de chlorure de méthylène, auxquels on ajoute 15 ml de chlorure de benzyle. La solution est chauffée à une température comprise entre 25 et 35°C pendant 25 heures. On ajoute alors 90 ml d'une solution à 10 % d'acétate de sodium dans le méthanol, filtre, lave au méthanol et sèche. 10g d'ester benzylique de l'héparine obtenu dans les conditions décrites ci-dessus sous forme de sel de sodium sont dissous dans 250 ml d'eau. A cette solution chauffée à 60°C environ on ajoute 0,9 g de soude. La température est maintenue 1h30 à 60°C environ, et le mélange réactionnel est ensuite refroidi vers 20°C et neutralisé par addition d'acide chlorhydrique dilué. On ajuste alors la concentration du milieu à 10 % en chlorure de sodium, et le produit est précipité dans 750 ml de méthanol, filtré et séché.

### Exemple 2 : Préparation du mélange M1

10 colonnes de verre de diamètre 100 mm et de hauteur 50 cm contenant chacune 4 litres de gel AcA 202 (gel sous forme de billes de polyacrylamide-agarose, de diamètre compris entre 60 et 140 am) sont utilisées en série pour le fractionnement :

Une solution contenant 30 g d'héparine dépolymérisée dans les conditions décrites dans l'exemple 1 est placée en tête de la première colonne et éluée par une phase mobile constituée d'une solution 0,2M de KH₂PO₄, à un débit de 300 ml/heure. Les fractions sont récoltées en fin de la dixième colonne.

Ce traitement permet de séparer efficacement les chaines oligosaccharidiques en fonction de leur masse moléculaire, et de les rassembler ensuite pour obtenir des mélanges ayant des caractéristiques désirées. Le mélange M1 a ainsi été obtenu, dont les caractéristiques physicochimiques et biologiques in vitro sont données dans le tableau 1.

### Exemple 3 : Préparation du mélange M2.

Le mélange M2 a été obtenu en suivant le même protocole de fractionnement que celui décrit dans l'exemple 2, en utilisant 50 g d'héparine dépolymérisée.

Les caractéristiques physicochimiques et biologiques in vitro du mélange M2 sont données dans le tableau 1.

### Exemple 4 : Préparation du mélange M5 et du mélange M6

Le mélange M5 a été obtenu en suivant le même protocole de fractionnement que celui décrit dans l'exemple 2, à partir de 30 g d'héparine dépolymérisée, mais en utilisant 10 colonnes de diamètre 9 cm et de hauteur 50 cm.

Le mélange M6 a été obtenu par un premier fractionnement réalisé selon le protocole de l'exemple 2, à partir de 150 g d'héparine dépolymérisée sur 10 colonnes de diamètre 18 cm et de hauteur 50 cm, avec un débit de phase mobile de 1,1 l/heure. Les fractions intéressantes ont été regroupées et les oligosaccharides précipités par addition de méthanol. Le solide recueilli a ensuite été mis en solution puis refractionné sur le même sytème chromatographique. Les fractions ont été recueillies en fin de la dixième colonne.

Les caractéristiques physicochimiques et biologiques in vitro des mélanges M5 et M6 sont données dans le tableau 1.

### Exemple 5 : Exemple comparatif : Préparation du mélange M3 et du mélange M4.

Le mélange M3 a été obtenu en suivant le même protocole de fractionnement que celui décrit dans l'exemple 2. Pour le mélange M4, après fractionnement selon le protocole de l'exemple 2, il a été obtenu par mélange d'une fraction sortant à 5530 daltons (63 %) et d'une fraction sortant à 7770 daltons (37 %).

Les caractéristiques physicochimiques et biologiques in vitro des mélanges M3 et M4 sont données dans le tableau 1.

### Exemple 6 : Etude de l'activité APTT in vitro sur plasma humain des mélanges de l'invention.

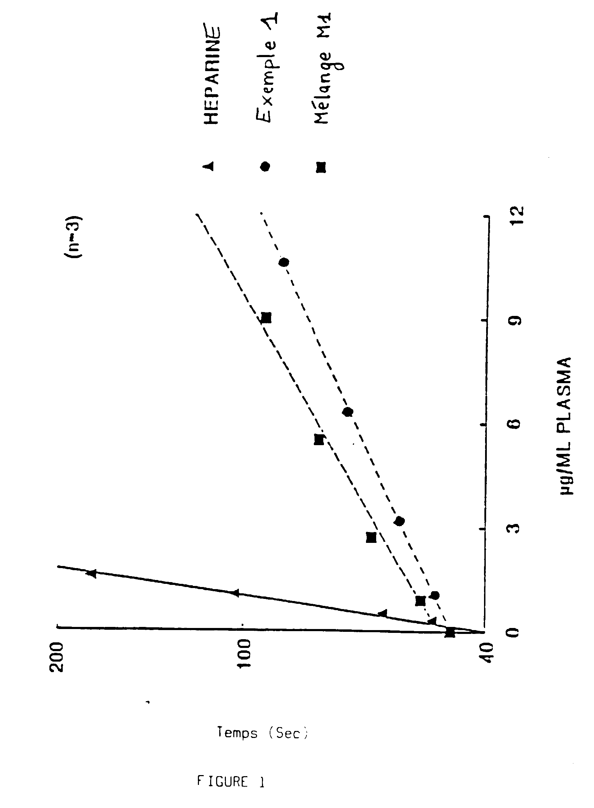

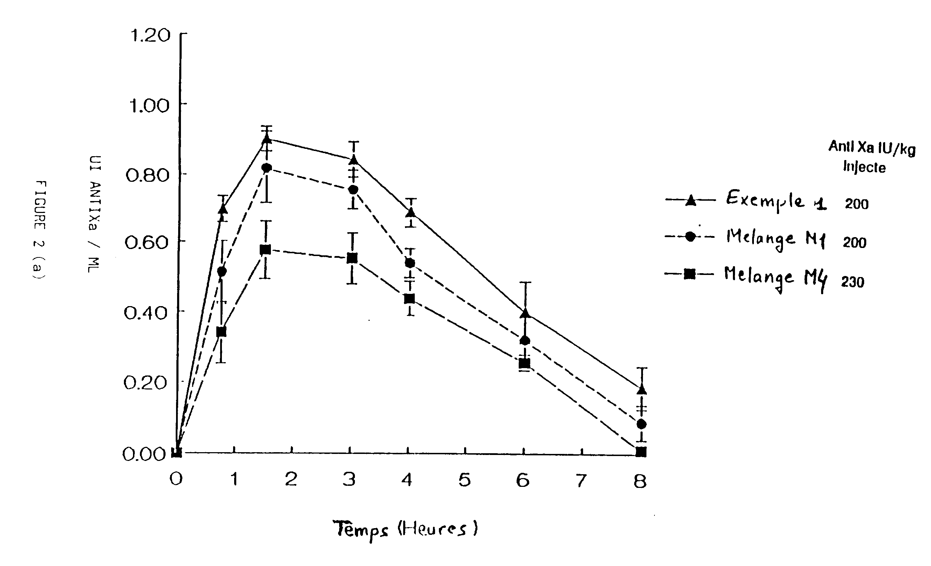

Les résultats obtenus sont présentés dans la figure 1. Ils montrent clairement que les mélanges de l'invention (mélange M1) font varier in vitro, sur plasma humain, l'APTT de manière beaucoup plus importante que l'héparine non fractionnée.

**Tableau 1**

| Produit | M1 | M2 | Ex. 1 | M3 | M4 | M5 | M6 |
|---|---|---|---|---|---|---|---|
| Masse moléculaire moyenne | 6410 | 6340 | 4280 | 5550 | 6360 | 6420 | 6780 |
| Répartition moléculaire (% MM) | | | | | | | |
| - > 7500 | 1 | 5,5 | 9 | 0 | 22 | 3,5 | 3,5 |
| - 7200±300 | 12,5 | 15 | 3,5 | 0 | 10,5 | 16,75 | 35,25 |
| - 6600±300 | 47 | 30,5 | 5 | 9 | 10 | 37,5 | 50,75 |
| - 6000±300 | 35 | 32,5 | 6,5 | 30 | 19,5 | 31,75 | 9,5 |
| - 5400±300 | 4,5 | 13,5 | 7,5 | 40,5 | 25 | 9,5 | 1 |
| - < 5100 | 0 | 3 | 68,5 | 20,5 | 13 | 1 | 0,1 |
| Activité in vitro | | | | | | | |
| - anti Xa | 109 | 127 | 94,25 | 116,6 | 130 | 126 | 106 |
| - anti IIa | 75 | 80 | 27,75 | 41,8 | 78 | 68 | 70 |

### Exemple 7 : Etude cinétique des activités anti Xa et anti IIa chez le lapin

Cette étude a été réalisée selon le protocole suivant :
- lapins : néo-zélandais (3,5 kg)
- volume injecté : voie sous-cutanée : 0,5 ml/kg (région abdominale)
- prises de sang : 3 ml de sang sont prélevés sur solution de citrate de sodium 3,1 % (1/10) au niveau de l'artère médiane de l'oreille aux temps 0 min, 45 min, 90 min, 3h, 4h, 6h, 8h.

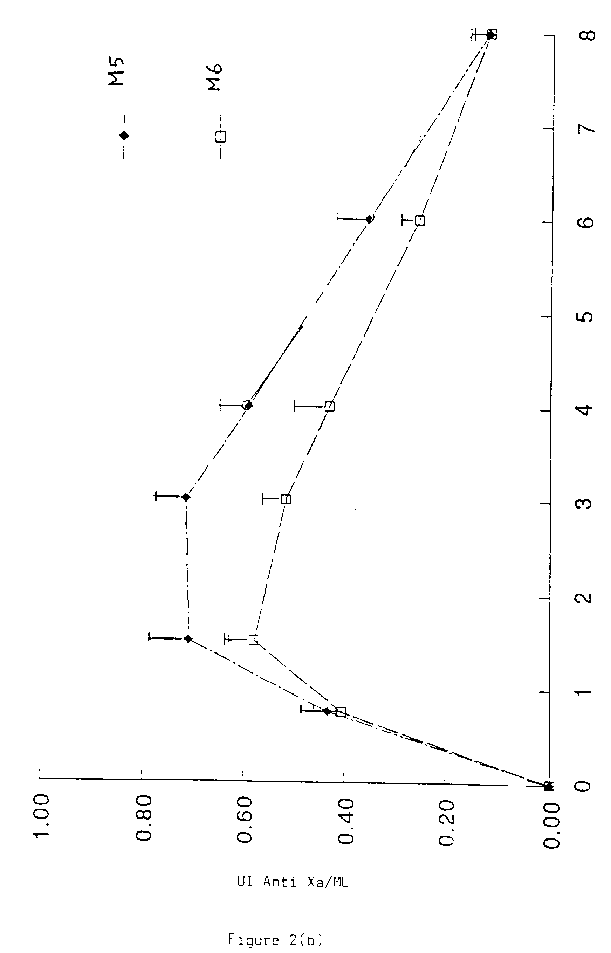

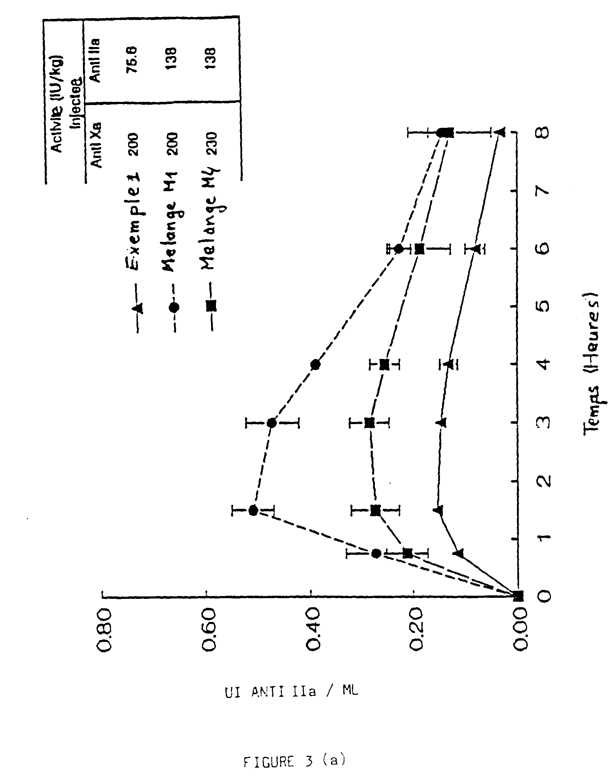

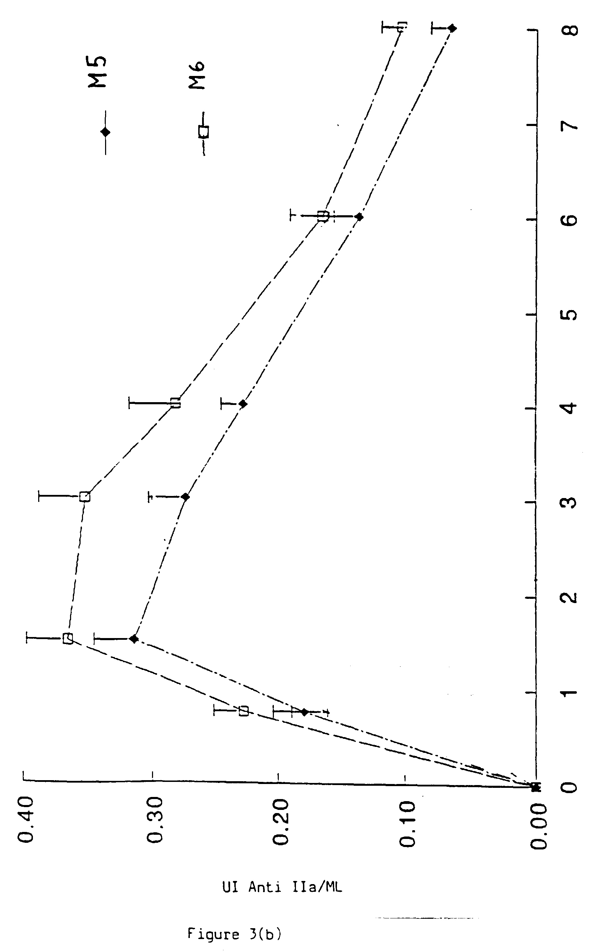

Après injection sous-cutanée au lapin des mélanges M1, M5 et M6 selon l'invention, ou d'héparine dépolymérisée selon l'exemple 1, à doses anti Xa égales, on obtient des activités anti Xa et anti IIa dans le plasma qui sont :
- parallèles et comparables pour l'anti Xa (figure 2),
- très supérieures avec le mélange de l'invention pour l'activité anti IIa (figure 3).

La même comparaison faite par rapport au mélange M4 décrit dans l'exemple 5 montre que les activités anti Xa du plasma demeurent parallèles mais que la différence est très importante pour l'activité anti IIa (figures 2 et 3).

Ces résultats montrent donc, de manière surprenante, que les mélanges de l'invention possèdent des activités anti Xa et anti IIa persistantes, soutenues et élevées in vivo. Ces mélanges sont de ce fait particulièrement avantageux par rapport à l'héparine non fractionnée, dont les activités anti Xa et anti IIa sont in vivo de très courte durée, mais également par rapport aux héparines de bas poids moléculaire décrites dans l'art antérieur, dont l'activité anti IIa in vivo est de très courte durée.

### Exemple 8 : Etude de l'activité in vivo des mélanges de l'invention, sur la thrombose veineuse chez le lapin après injection intra-veineuse.

Le tableau 2 rassemble les résultats obtenus. Ce tableau montre que le mélange M3 (exemple 4), dont la répartition moléculaire est différente de celle revendiquée, et l'héparine dépolymérisée (exemple 1) sont moins actifs que le mélange M1 de l'invention. En particulier, celui-ci donne une activité anti IIa nettement supérieure.

### Exemple 9 : Etude de l'activité in vivo des mélanges de l'invention, sur la thrombose veineuse chez le lapin après injection sous-cutanée.

Les tableaux 3 et 4 rassemblent les résultats obtenus. Ces tableaux montrent que les mélanges M1, M5 et M6 sont plus actifs qu'une héparine dépolymérisée de l'art antérieur (exemple 1), et qu'ils présentent une durée d'action au moins équivalente.

### Exemple 10 : Etude de l'activité in vivo sur le temps de saignement chez le rat.

Les résultats présentés dans le tableau 5 suivant montrent clairement que les mélanges de l'invention (illustrés par les mélanges M1, M5 et M6) font moins saigner que les autres héparines de faible masse moléculaire. Les résultats donnés correspondent à l'augmentation du temps de saignement en minutes par rapport à des valeurs contrôle. A titre comparatif, à 2 mg/kg, l'héparine induit une augmentation du temps de saignement de 13.

**Tableau 5**

| Dose\Produit | M1 | M5 | M6 | Ex. 1 | M4 |
|---|---|---|---|---|---|
| 4 mg/kg | 1,07±0,4 | | | 4±1,1 | 8,6±3.6 |
| 8 mg/kg | 3,0±0,7 | 4,0±0,5 | 9,6±1,8 | 7,9±2,1 | 33,7±8 |

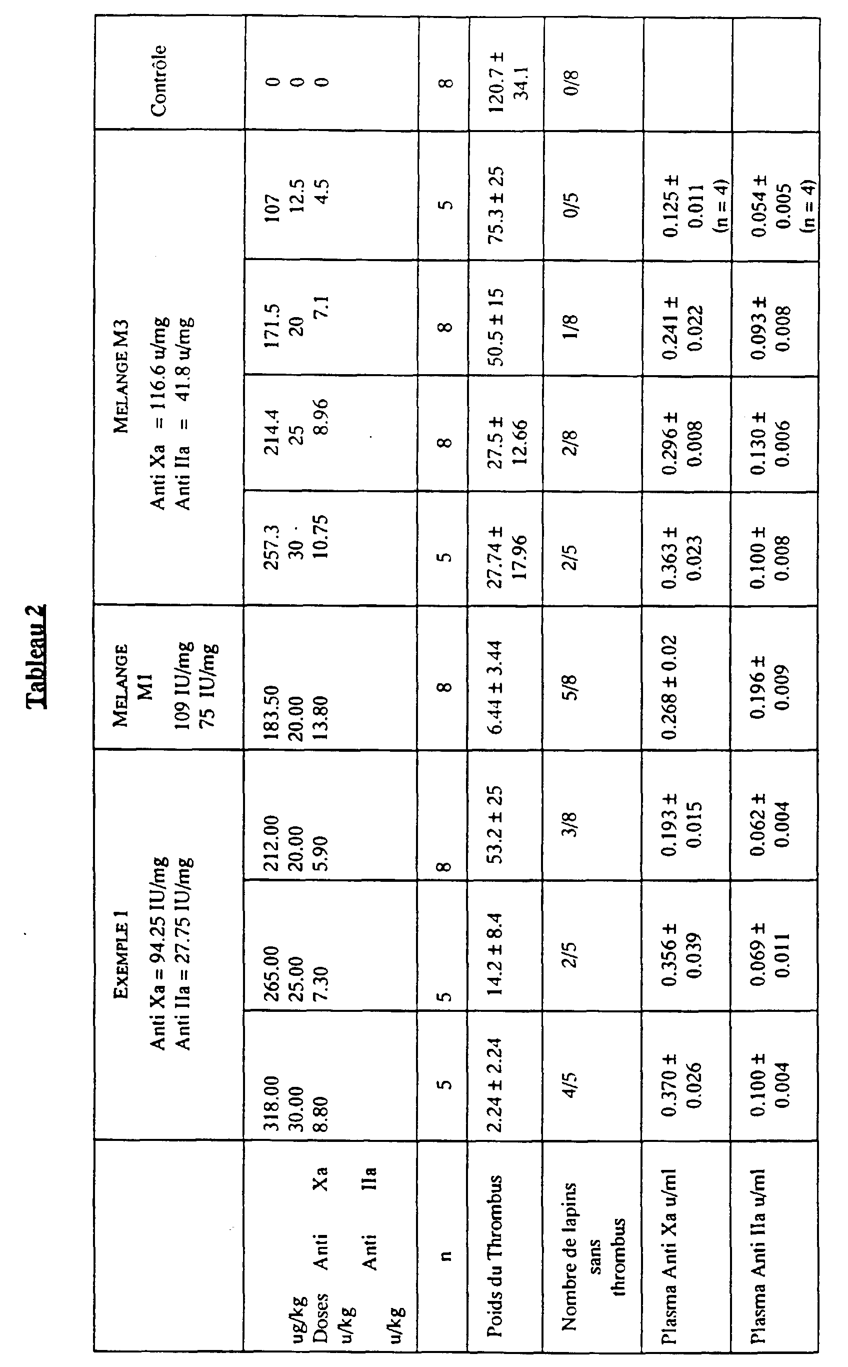

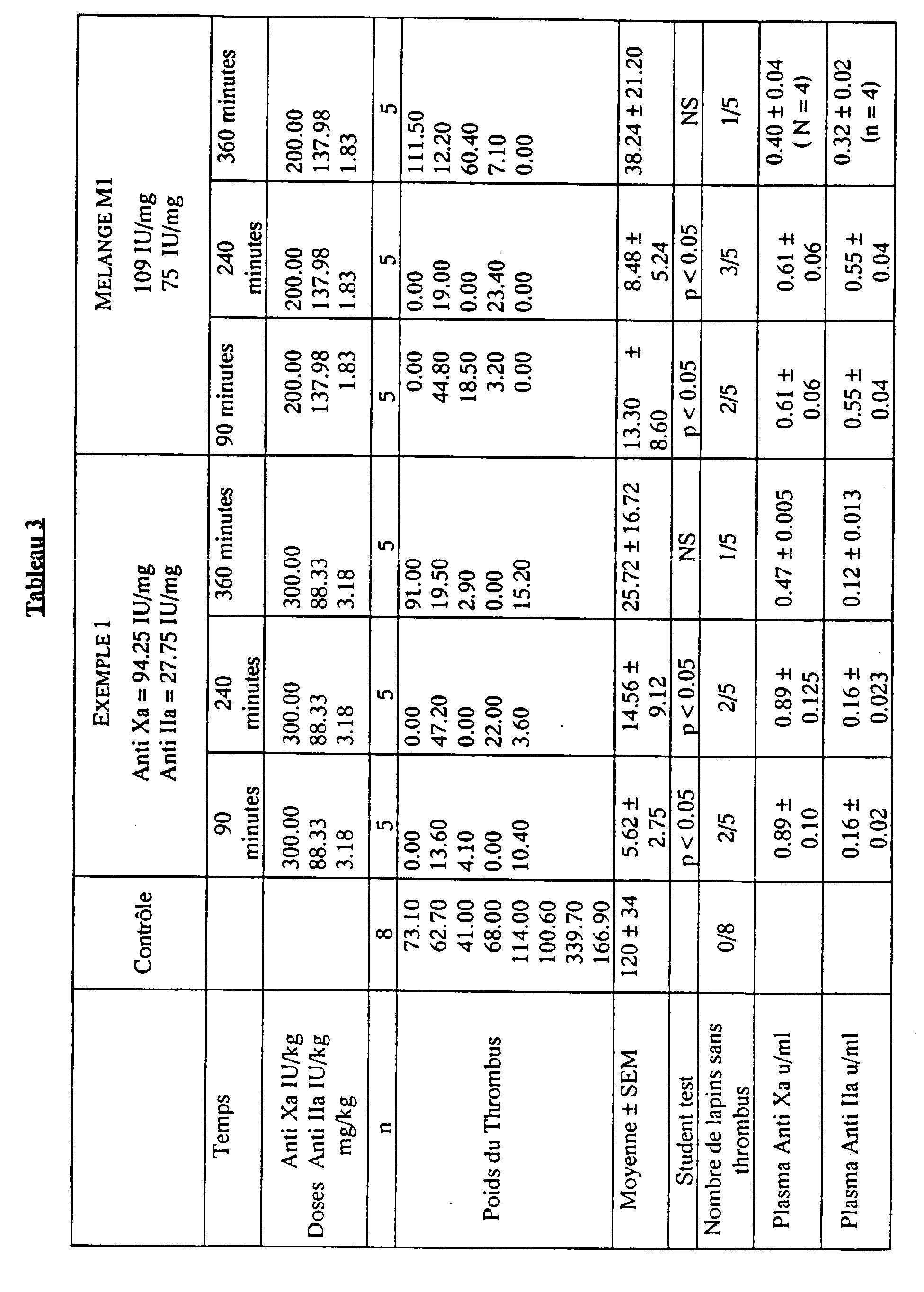

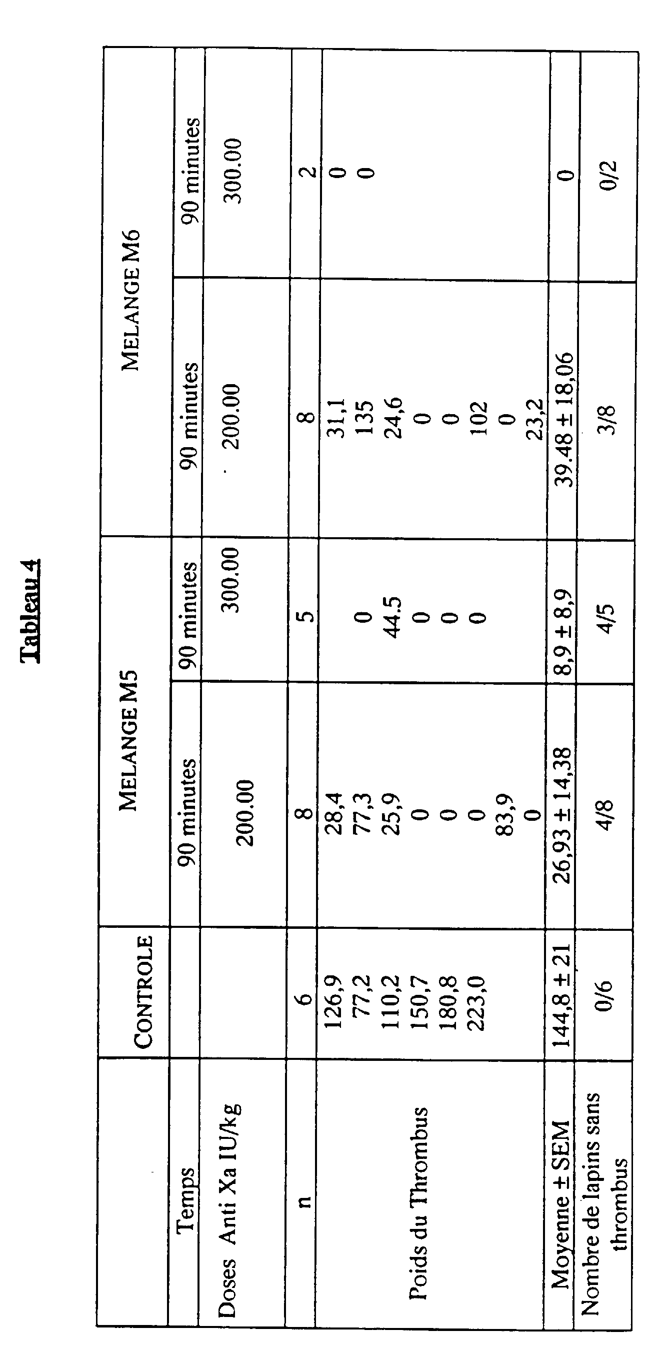

## Revendications

1. Mélange d'oligosaccharides sulfatés présentant la structure générale des oligosaccharides constitutifs de l'héparine, caractérisé en ce qu'il comprend au moins 70 % d'oligosaccharides ayant une masse moléculaire comprise entre 5400 et 7800 daltons, au moins 5 % d'oligosaccharides ayant une masse moléculaire supérieure à 6900 daltons, et en ce qu'il possède une activité anti IIa supérieure à 60 UI/mg (méthode de Anderson L.O. et al., Thrombosis research 15 (1979) 531-541, avec le WHO LMWH1 comme étalon).

2. Mélange selon la revendication 1 caractérisé en ce qu'il comprend au moins 70 % d'oligosaccharides ayant une masse moléculaire comprise entre 5700 et 7500 daltons.

3. Mélange selon la revendication 2 caractérisé en ce qu'il comprend au moins 60 % d'oligosaccharides ayant une masse moléculaire comprise entre 5700 et 6900 daltons.

4. Mélange selon l'une des revendications 1 à 3 caractérisé en ce qu'il comprend au moins 10 % d'oligosaccharides ayant une masse moléculaire supérieure à 6900 daltons.

5. Mélange selon l'une des revendications 1 à 4 caractérisé en ce qu'il possède une activité anti IIa égale ou supérieure à 70 UI/mg.

6. Mélange selon l'une des revendications 1 à 5 caractérisé en ce qu'il s'agit d'une fraction d'héparine.

7. Mélange selon l'une des revendications 1 à 5 caractérisé en ce qu'il s'agit d'une fraction d'héparine dépolymérisée.

8. Mélange selon la revendication 7 caractérisé en ce qu'il est constitué d'oligosaccharides ayant un acide 2-O-sulfo 4 énopyranosuronique à l'une de leurs extrêmités.

9. Mélange selon la revendication 8 caractérisé en ce qu'il s'agit d'une fraction d'héparine dépolymérisée par action d'une base sur un ester d'héparine.

10. Procédé de préparation d'un mélange selon l'une des revendications 1 à 9 caractérisé en ce que l'on fractionne une héparine ou une héparine dépolymérisée par gel-filtration, ce fractionnement comprenant successivement les étapes consistant à (i) mettre l'héparine ou l'héparine dépolymérisée de départ en solution dans l'éluant, (ii) faire passer la solution ainsi obtenue à travers une colonne au moins contenant le support solide pour la gel-filtration, préalablement équilibrée avec le même éluant, et (iii) récupérer les fractions de poids moléculaire désiré.

11. Procédé selon la revendication 10 caractérisé en ce que l'on utilise une héparine dépolymérisée.

12. Procédé selon la revendication 11 caractérisé en ce que l'on utilise une héparine dépolymérisée par action d'une base sur un ester d'héparine.

13. Procédé selon la revendication 10 caractérisé en ce que, lors de la seconde étape du fractionnement, le support est réparti en plusieurs colonnes disposées en série.

14. Procédé selon la revendication 10 caractérisé en ce que la seconde étape est réalisée en utilisant successivement 2 types de supports au moins, ayant des caractéristiques de séparation différentes.

15. Composition pharmaceutique ayant comme principe actif un mélange d'oligosaccharides selon l'une quelconque des revendications 1 à 9.

16. Composition pharmaceutique selon la revendication 15 destinée au traitement et à la prévention des thromboses veineuses et artérielles.

17. Composition pharmaceutique selon la revendication 15 destinée à la prévention des accidents thrombotiques artériels, notamment dans le cas d'infarctus du myocarde.

18. Composition pharmaceutique selon la revendication 15 destinée à une utilisation en régime post-opératoire, dans la prévention de thromboses veineuses chez les malades chirurgicaux.

19. Utilisation d'un mélange d'oligosaccharides selon l'une quelconque des revendications 1 à 9 dans la prévention des thromboses dans le matériel chirurgical.

## Claims

1. Mixture of sulphated oligosaccharides having the general structure of the constituent oligosaccharides of heparin, characterized in that it comprises at least 70 % of oligosaccharides having a molecular weight between 5,400 and 7,800 daltons, at least 5 % of oligosaccharides having a molecular weight greater than 6,900 daltons, and in that it has an anti-IIa activity greater than 60 IU/mg (method of Anderson L.O. et al., Thrombosis Research, 15 (1979), 531-541, with WHO LMWH1 as standard).

2. Mixture according to claim 1, characterized in that it comprises at least 70 % of oligosaccharides having a molecular weight between 5,700 and 7,500 daltons.

3. Mixture according to claim 2, characterized in that it comprises at least 60 % of oligosaccharides having a molecular weight between 5,700 and 6,900 daltons.

4. Mixture according to one of claims 1 to 3, characterized in that it comprises at least 10 % of oligosaccharides having a molecular weight greater than 6,900 daltons.

5. Mixture according to one of claims 1 to 4, characterized in that it has an anti-IIa activity equal to or greater than 70 IU/mg.

6. Mixture according to one of claims 1 to 5, characterized in that it is a fraction of heparin.

7. Mixture according to one of claims 1 to 5, characterized in that it is a fraction of depolymerized heparin.

8. Mixture according to claim 7, characterized in that it consists of oligosaccharides having a 2-O-sulpho-4-enopyranosuronic acid at one of their ends.

9. Mixture according to claim 8, characterized in that it is a heparin fraction which has been depolymerized by the action of a base on a heparin ester.

10. Process for the preparation of a mixture according to one of claims 1 to 9, characterized in that a heparin or a depolymerized heparin is fractionated by gel filtration, this fractionation successively comprising steps consisting in (i) dissolving the starting heparin or depolymerized heparin in the eluent, (ii) passing the solution thus obtained through a column containing at least the solid support for the gel filtration which has been equilibrated beforehand with the same eluent, and (iii) recovering the fractions of desired molecular weight.

11. Process according to claim 10, characterized in that a depolymerized heparin is used.

12. Process according to claim 11, characterized in that a heparin is used which has been depolymerized by the action of a base on a heparin ester.

13. Process according to claim 10, characterized in that, during the second step of the fractionation, the support is distributed in several columns arranged in series.

14. Process according to claim 10, characterized in that the second step is performed by successively using at least 2 types of support, having different separation characteristics.

15. Pharmaceutical composition having as active principle a mixture of oligosaccharides according to any one of claims 1 to 9.

16. Pharmaceutical composition according to claim 15, which is intended for the treatment and for the prevention of venous and arterial thrombosis.

17. Pharmaceutical composition according to claim 15, which is intended for the prevention of arterial thrombotic accidents, in particular in the case of myocardial infarction.

18. Pharmaceutical composition according to claim 15, which is intended for use in post-operative care, in the prevention of venous thrombosis in surgical patients.

19. Use of a mixture of oligosaccharides according to any one of claims 1 to 9 in the prevention of thrombosis in the surgical equipment.

## Patentansprüche

1. Mischung von sulfatierten Oligosacchariden, die die allgemeine Struktur der wesentlichen Oligosaccharide des Heparins aufweisen, dadurch gekennzeichnet, daß sie mindestens 70 % Oligosaccharide mit einer Molmasse zwischen 5400 und 7800 Dalton, mindestens 5 % Oligosaccharide mit einer Molmasse von höher als 6900 Dalton umfaßt, und dadurch, daß sie eine Aktivität anti IIa von höher als 60 IE/mg besitzt [Methode von Anderson L.O. et al., Thrombosis research 15 (1979) 531-541, mit WHO LMWH1 als Standard].

2. Mischung nach Anspruch 1, dadurch gekennzeichnet, daß sie mindestens 70 % Oligosaccharide mit einer Molmasse zwischen 5700 und 7500 Dalton umfaßt.

3. Mischung nach Anspruch 2, dadurch gekennzeichnet, daß sie mindestens 60 % Oligosaccharide mit einer Molmasse zwischen 5700 und 6900 Dalton umfaßt.

4. Mischung nach einem der Ansprüche 1 bis 3, dadurch gekennzeichnet, daß sie mindestens 10 % Oligosaccharide mit einer Molmasse von höher als 6900 Dalton umfaßt.

5. Mischung nach einem der Ansprüche 1 bis 4, dadurch gekennzeichnet, daß sie eine Aktivität anti IIa von gleich oder höher als 70 IE/mg besitzt.

6. Mischung nach einem der Ansprüche 1 bis 5, dadurch gekennzeichnet, daß es sich um eine Fraktion von Heparin handelt.

7. Mischung nach einem der Ansprüche 1 bis 5, dadurch gekennzeichnet, daß es sich um eine depolymerisierte Fraktion von Heparin handelt.

8. Mischung nach Anspruch 7, dadurch gekennzeichnet, daß sie aus Oligosacchariden besteht, die an einem ihrer Enden eine 2-O-Sulfo-4-enopyranosuronsäure besitzen.

9. Mischung nach Anspruch 8, dadurch gekennzeichnet, daß es sich um eine Fraktion von Heparin handelt, die durch Einwirkung einer Base auf einen Heparinester depolymerisiert wurde.

10. Verfahren zur Herstellung einer Mischung nach einem der Ansprüche 1 bis 9, dadurch gekennzeichnet, daß man ein Heparin oder ein depolymerisiertes Heparin durch Gelfiltration fraktioniert, wobei diese Fraktionierung nacheinander die folgenden Stufen umfaßt:
(i) das Heparin oder das depolymerisierte Heparin als Ausgangsstoff in Lösung eines Eluierungsmittels zu bringen,
(ii) die auf diese Weise erhaltene Lösung über eine Kolonne passieren zu lassen, die mindestens den festen Träger für die Gelfiltration enthält, der zuvor mit dem gleichen Eluierungsmittel ins Gleichgewicht gebracht wurde, und
(iii) die Fraktionen mit dem gewünschten Molekulargewicht zu gewinnen.

11. Verfahren nach Anspruch 10, dadurch gekennzeichnet, daß man eine depolymerisierte Fraktion von Heparin verwendet.

12. Verfahren nach Anspruch 11, dadurch gekennzeichnet, daß man eine Fraktion von Heparin verwendet, die durch Einwirkung einer Base auf einen Heparinester depolymerisiert wurde.

13. Verfahren nach Anspruch 10, dadurch gekennzeichnet, daß bei der zweiten Stufe der Fraktionierung der Träger in mehrere, in Reihe angeordnete Kolonnen aufgeteilt ist.

14. Verfahren nach Anspruch 10, dadurch gekennzeichnet, daß die zweite Stufe unter Verwendung von nacheinander mindestens zwei Trägertypen realisiert wird, die unterschiedliche Trennungs-Charakteristiken aufweisen.

15. Pharmazeutische Zusammensetzung, die als Wirkstoff eine Mischung von Oligosacchariden nach irgendeinem der Ansprüche 1 bis 9 aufweist.

16. Pharmazeutische Zusammensetzung nach Anspruch 15, vorgesehen für die Behandlung und Vorbeugung von venösen und arteriellen Thrombosen.

17. Pharmazeutische Zusammensetzung nach Anspruch 15, vorgesehen für die Vorbeugung von arteriellen thrombotischen Zwischenfällen, insbesondere im Fall des Myokardinfarktes.

18. Pharmazeutische Zusammensetzung nach Anspruch 15, vorgesehen für eine Verwendung im post-operativen Bereich zur Vorbeugung von venösen Thrombosen bei chirurgisch Kranken.

19. Verwendung einer Mischung von Oligosacchariden nach irgendeinem der Ansprüche 1 bis 9 für die Vorbeugung von Thrombosen in chirurgischem Material.
